# EUROPEAN PATENT SPECIFICATION

(11) **EP 4 419 200 B1**
(45) Date of publication and mention of the grant of the patent: **13.05.2026**
(21) Application number: 22800369.5
(22) Date of filing: 20.10.2022
(51) Int. Cl.: A61N 5/10

(54) **SYSTEMS AND COMPUTER SOFTWARE FOR OPTIMIZED RADIATION THERAPY**
SYSTEME UND COMPUTERSOFTWARE FÜR OPTIMIERTE STRAHLENTHERAPIE
SYSTÈMES ET LOGICIEL INFORMATIQUE POUR UNE RADIOTHÉRAPIE OPTIMISÉE

(30) Priority: 22.10.2021 US 202163270855 P
(43) Date of publication of application: 28.08.2024
(73) Proprietor: ViewRay Systems, Inc., Denver, Colorado 80206 (US)
(72) Inventor: DEMPSEY, James F., Denver, Colorado 80202 (US); KAWRYKOW, Iwan, Denver, Colorado 80202 (US)
(74) Representative: Carroll, Christopher P.
(86) International application number: PCT/IB2022/060069
(87) International publication number: WO 2023/067529

(56) References cited:
- EP-A2- 1 725 166
- EP-B1- 1 725 166
- US-A1- 2016 228 728
- US-A1- 2017 252 577
- US-A1- 2018 369 611
- US-A1- 2021 228 907

## Description

### DESCRIPTION OF THE RELATED ART

Radiation therapy involves delivering radiation dose to a patient at a specific location to treat tissue, most typically cancerous tissue or tumors. To deliver radiation mainly to the target tissue and avoid irradiating healthy tissue, radiation beams are shaped and delivered in particular sequences and from particular directions according to a radiation treatment plan. In particular, optimization processes are used to mathematically determine the best radiation delivery parameters for the radiation treatment plan based on a radiation prescription determined by clinicians, patient imaging, radiotherapy device capabilities, etc. In some cases, the delivery of radiation can be guided by imaging prior to and/or during therapy and treatment plans can be improved through reoptimization. US 2016/228728A1 describes Magnetic resonance (MR) guided radiation therapy (MRgRT) that enables control over the delivery of radiation based on patient motion indicated by MR imaging (MRI) images captured during radiation delivery. A method for MRgRT includes: simultaneously using one or more radiation therapy heads to deliver radiation and an MRI system to perform MRI; using a processor to determine whether one or more gates are triggered based on at least a portion of MRI images captured during the delivery of radiation; and in response to determining that one or more gates are triggered based on at least a portion of the MRI images captured during the delivery of radiation, suspending the delivery of radiation. EP1725166A2 discloses a device and a process for performing high temporal and spatial resolution MR imaging of the anatomy of a patient during intensity modulated radiation therapy (IMRT) to directly measure and control the highly conformal ionizing radiation dose delivered to the patient for the treatment of diseases caused by proliferative tissue disorders. This invention combines the technologies of open MRI, multileaf-collimator or compensating filter-based IMRT delivery, and cobolt teletherapy into a single co-registered and gantry mounted system. US 2018/369611A1 describes systems and methods for adapting and/or updating radiotherapy treatment plans based on biological and/or physiological data and/or anatomical data extracted or calculated from imaging data acquired in real-time (e.g., during a treatment session). Functional imaging data acquired at the time of radiation treatment is used to modify a treatment plan and/or dose delivery instructions to provide a prescribed dose distribution to patient target regions. Also described are methods for evaluating treatment plans based on imaging data acquired in real-time. A further relevant document is formed by US 2016/228728 A1.

### SUMMARY

The invention is set out in the appended set of claims. A machine-readable medium encoded with the computer software relating to improved radiotherapy planning and treatment is disclosed. Methods of treatment of a patient mentioned hereinafter do not form part of the present invention. In one aspect, imaging parameters can be automatically recalled from computer memory for controlling an MRI system to perform treatment-day scans of a patient prior to treatment. The treatment-day scans can then be automatically initialized and the MRI system can be controlled to perform the treatment-day scans according to the recalled imaging parameters. A reoptimized radiation treatment plan can then be automatically generated, and predicted doses to anatomical structures of the patient based on the reoptimized radiation treatment plan can be displayed. In some implementations, prescription doses to anatomical structures can be displayed concurrently with the displaying of predicted doses. Finally, a radiation therapy device can be controlled to deliver radiation according to a selected radiation treatment plan.

In some variations, the automatic initialization of the treatment-day scans can be based on a sensor indicating that a door to a treatment room is closed or the patient being sensed as being positioned at isocenter.

In some embodiments, the disclosed systems, methods, and software can provide for additional treatment plan reoptimization, including generation of a new reoptimized radiation treatment plan or modification of one of the automatically generated reoptimized radiation treatment plane(s). Furthermore, the disclosed systems, methods and software can enable clinicians to perform different reoptimization tasks simultaneously including through parallel workflow interfaces. This can include, for example, enabling multiple clinicians to perform autocontouring simultaneously or enabling clinicians to attempt different reoptimization strategies simultaneously. In yet other variations, an imaging plane can be moved based on user input and a reoptimized radiation treatment plan can be updated based on a new location of the imaging plane. Certain embodiments also allow for the receipt of user input modifying tracking parameters, dose parameters, structure shapes, or boundary parameters utilized by the system when controlling the radiation therapy device to deliver the radiation.

### BRIEF DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings,
Figure 1 is a simplified block diagram of an exemplary system for radiation therapy planning and treatment delivery in accordance with certain aspects of the present disclosure,
Figure 2 is a flowchart of an exemplary software-implemented process for radiation therapy planning and treatment delivery in accordance with certain aspects of the present disclosure,
Figure 3 is a flowchart of an exemplary software-implemented process for automatic radiation treatment plan reoptimization in accordance with certain aspects of the present disclosure, and
Figure 4 is a flowchart of exemplary reoptimization tasks that can be performed in parallel by the disclosed software in accordance with certain aspects of the present disclosure.

### DETAILED DESCRIPTION

Conventional radiation therapy planning and delivery consists of several phases. Initially, the patient is imaged (e.g., with MRI and/or CT) to make a record of the patient's anatomy and identify target locations (e.g., tumors or regions of potential microscopic disease) for radiation treatment. The images are then reviewed by clinicians and a radiation treatment plan is determined. When it is time to treat the patient (which may be days or weeks later) the patient goes to a treatment facility and receives treatment according to the radiation treatment plan.

A simplified description of a radiation treatment plan is a sequence of operations performed by a radiation delivery device, typically in conjunction with an imaging system, to provide a prescribed dose to various locations of a patient's anatomy. A radiation treatment plan can include, for example, specifying angles of delivery for a radiation beam, beam-on times, beam collimator settings, patient couch positions, settings for imaging devices used during treatment delivery, etc. While the above is intended to provide a simplified introduction to radiation treatment plans, it is understood that an actual radiation treatment plans can have other aspects not specifically enumerated.

Adaptive radiation treatment provides for the changing or additional refinement of a radiation treatment plan based on current conditions of the patient. For example, if the patient gained or lost weight between the initial imaging and treatment day, the shape and location of the patient's healthy organs and targets can change from the original treatment plan. This can mean, for example, that the position of the treatment couch may need to be adjusted and the treatment plan may need to be reoptimized in order to deliver a clinically acceptable treatment plan.

Prior to the present disclosure, an adaptive radiation treatment required the physical presence of clinical team members such as physicians, physicists, dosimetrists, and therapists at the console of the radiation therapy system in a lengthy procedure to reoptimize a plan. For example, control of the therapy system was manually driven by a human operators performing the setting and acquiring of treatment day imaging, manually performing adaptive computations and performing evaluations of alternative treatment plans. Clinicians with different roles in the process, e.g., physicists and physicians, took turns reviewing and performing tasks required in an entirely sequential fashion, requiring coordination between them to perform the tasks. Many system settings and therapy decisions had to be made under time pressure with the patient waiting on the treatment couch.

As used herein, the terms "clinician," "physician," and the like, are provided to illustrate that many embodiments of the present disclosure are contemplated for use in a clinical setting to treat medical conditions of various patients. However, such terms should not be considered limiting and instead considered equivalent with the generic term "user," as the disclosed software can be utilized by any person.

As described in greater detail below, the present disclosure describes numerous image guided radiotherapy system and computer implemented processes that allow for improvements in adaptive radiotherapy processes thereby improving dose delivery, safety, efficiency and convenience in numerous processes.

Initial and pretreatment imaging scans can utilize any number and combination of different imaging types to characterize the patient for the delivery of radiotherapy. In some embodiments, the imaging types can include, for example, one or more of 2D planar, volumetric, 4D volumetric, and real-time 4D volumetric. 2D planar generally refers to a substantially planar slice through a patient (e.g., on axial, sagittal or coronal planes). Volumetric imaging generally refers to constructing a 3D patient volume depicting a region of the patient. 4D volumetric generally refers to volumetric (3D) images of the patient over time. For example, a 4D volumetric image can capture a patient volume at different times corresponding to a patient breathing cycle. In some implementations, 4D imaging can document breathing-cycle intervals, for example, as binned from some measure of the breathing cycle such as chest height or spirometer air volume. As such, the "times" for 4D volumetric imaging may not be a function of actual time. In contrast, "real-time" 4D volumetric imaging generally refers to 4D volumetric imaging that is reconstructed (and optionally provided to clinicians or made available to the radiation therapy system) in real time. As used herein, "real-time" means with minimal delay between image data capture and image development. In particular, the acquisition, reconstruction, and processing time can be less than the frame rate so that the imaging can keep pace with the procedure. In this way, the utilization of such real-time images can inform the clinician or the system about the nearly current state of the patient. In practice, implementations of real-time imaging can involve delays of less than one second and sometimes much less, e.g., 0.25 seconds to reconstruct and display a volumetric image. The required frame rate can depend on the speed of the motions being tracked in the patient, with 0.25 seconds being sufficient to resolve typical cardiac and respiratory motions.

The imaging needed to characterize patient anatomy for use on treatment day can be described with regard to what is referred to herein as "imaging parameters." Examples of imaging parameters can include contrast, dimensionality, frame rate, resolution, field of view, order of scanning, etc. Specific examples of the above can include, for example, contrast (e.g., T1/T2), dimensionality (e.g., 3D, 4D, real-time 4D), frame rate (e.g., 10 Hz, 4 Hz, 2 Hz), resolution (e.g., 0.5 mm, 1.0 mm, 2.0 mm), field of view (e.g., 30 cm DSV, 50 cm DSV, 75 cm DSV), order of scanning (e.g., any desired order of scanning axial, sagittal and coronal planes to generate images corresponding to any of the above-described imaging types), etc.

With the determination and acquisition of desired imaging parameters, the imaging parameters can be stored in a database or other computer system for recall at any time. For example, as described herein, the imaging parameters can be automatically recalled at a particular time on treatment day to have the needed imaging sequences prepared for execution with the patient before (or during) patient treatment.

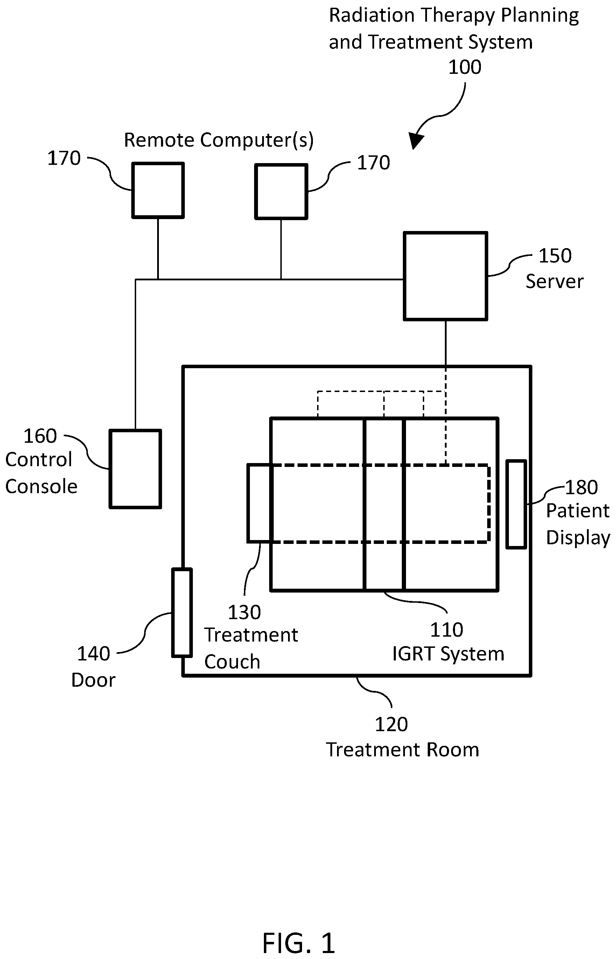

Figure 1 is a simplified block diagram of an exemplary radiation therapy planning and treatment system. As depicted, the radiation therapy planning and treatment system 100 can include an image guided radiation therapy (IGRT) system 110 located inside treatment room 120. Examples of IGRT systems can include medical linear accelerators (linacs), particle (protons, neutrons, etc.) therapy systems, or electron beam systems. Such systems can be guided by imaging systems such as MRI, CT, etc. The example imaging system shown depicts an open or split MRI where the radiation beam (e.g., delivered by a linac) is between the halves of the open MRI. The IGRT system may also include or incorporate a treatment couch 130 to support the patient within the IGRT system during treatment. The treatment couch can be configured to be adjustable in up to three dimensions (e.g., X, Y, Z) to position the patient at a desired location.

Treatment room 120 can be a vault or other enclosure that may be radiation shielded to prevent other portions of the facility from radiation generated by the radiation therapy device. The treatment room can also include magnetic and/or RF shielding to prevent external interference with the IGRT system and to prevent magnetic fields or RF from the IGRT system from escaping. Door 140 can be utilized to allow access to the treatment room. Such a door can have shielding similar to the treatment room to prevent the effects described above. As further described herein, door 140 can also include interlocks or other sensors to detect whether the treatment room door is closed.

One or more computing systems, diagrammatically simplified as server 150, can control the IGRT system and treatment couch. The server 150 can be located within the treatment facility or at a remote location. The server may be in wired and/or wireless communication with IGRT system 110, treatment couch 130, and/or more computers, e.g., control console 160 and one or more remote computers 170. In some embodiments, the server can be configured to operate as a web server and thus have the ability to cause the display of one or more radiation treatment plans or treatment delivery information at any combination of the control console and remote computers. Such displays of radiation treatment plans or other data from the server can be synchronized and in real-time thereby allowing users to interactively and collaboratively edit and/or approve radiation treatment plans. In certain embodiments, the server and software running thereon can require establishment of a secure connection before transmitting and/or receiving data relating to any of the operations described herein.

Control console 160 can be a workstation or other computing terminal that may be utilized by a clinician to control operation of the IGRT system. In some embodiments, the console can be proximate to the treatment room to allow the clinician providing treatment access to the IGRT system and the patient. In contrast, remote computer(s) 170 can be any connected computing system, for example, computers utilized by clinicians such as radiation oncologists or medical physicists that may not necessarily be proximate to the treatment room. Examples of remote computers can include, for example, desktop computers, notebook computers, tablet computers, smart phones, etc.

A patient display 180 can be included in some embodiments to allow the patient to observe the progress of treatment and allow them to assist in the positioning of their body during therapy. A patient display can be, for example, a computer screen or a visual projection onto a viewing surface or video-enabled goggles. Such screens or surfaces can be disposed within the MRI bore, or may be located outside the MRI system, but still viewable by the patient.

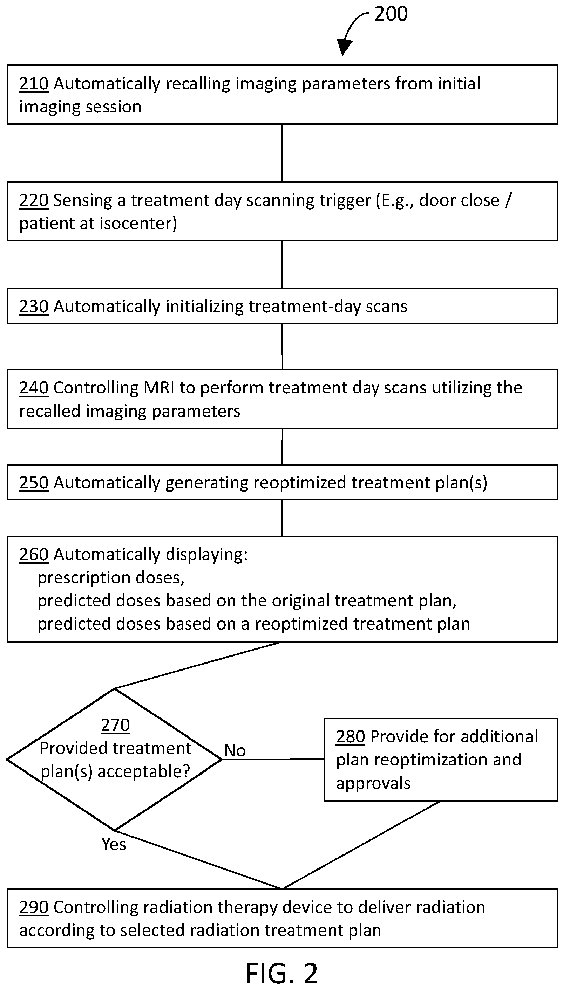

Figure 2 is a flowchart of an exemplary process for radiotherapy implemented by software in conjunction with an image guided radiotherapy system. Process 200 is depicted as an example showing a number of features of the disclosed software, however, not every element of process 200 nor the depicted order is required.

Patient treatment can begin by the patient being brought to the treatment room and placed on the treatment couch. Clinicians may then secure the patient to prevent movement and thereby help ensure accurate delivery of radiation. The system may then proceed to automatically position the treatment couch at the appropriate location within the IGRT system. In some cases, this can include the treatment couch being translated into an imaging system such as an MRI. In other cases, such as for radiation sources utilizing a robotic arm, the treatment couch may remain substantially stationary.

Embodiments of the present disclosure can reduce the overall time to safely treat a patient by utilizing the disclosed software processes, such as the example process 200 in Figure 2. This process can include, at 210, automatically recalling imaging parameters from computer memory (e.g., on server 150). These imaging parameters can be utilized (e.g., at 240, below) for controlling an MRI system to perform treatment-day scans of the patient on the treatment couch prior to treatment. The imaging parameters can include those determined from the process of obtaining the initial scans as described above. The imaging parameters can be loaded into the software that will perform treatment-day scans, such as server 150. The imaging parameters can optionally be displayed at control console 160 or any other connected computing system, such as remote computers 170.

Patient treatment can be sped up by the system automatically initializing the treatment-day scans based on sensing a treatment-day scanning trigger. For example, in certain embodiments, automatically initializing can be based on a sensor indicating that a door to the treatment room is closed. For example, there may be mechanical, electrical, or optical sensors that detect the closure and/or locking of the door to the treatment room. The output of such sensors can be converted to an electrical signal or status in a software system that can be utilized by the disclosed processes as a trigger to execute the initialization of the treatment-day scans.

In other embodiments, the automatic initializing can be based on sensing that the patient is at isocenter. For example, various IGRT systems can include sensors such as lasers, cameras, etc., that can detect the position of the patient relative to the isocenter. Such detection and analysis can be performed by image analysis, time-of-flight measurements, phase-shift translation registration algorithms, etc. For example, a phase shift translation registration algorithm can perform analysis of imaging of the patient taken during patient positioning and determine a best shift in the patient to align portions of the patient anatomy (e.g., a target tumor) to what it was in the initial imaging scans. In some embodiments, this can include limiting the field of view of these imaging scans to a fairly small region (e.g., 2-3 cm) in order to not skew a determined shift as a result of changes in other patient anatomy such as nearby organs. Once a needed shift is determined, in some cases, the patient may be asked to or assisted in physically moving to a location more proximate to isocenter. However, in other embodiments, the patient couch may be controlled by a computer to move the patient to isocenter based on the determined needed shift. In various embodiments, process steps 210 and 220 can be reversed. This means, for example, that the automatic recall of imaging parameters may not occur until the patient is ready for treatment as determined by a closed door to the treatment room or a determination of the patient is at isocenter.

The process may include, at 230, automatically initializing the treatment-day scans, for example after the sensing of a treatment-day scanning trigger as discussed above. The treatment-day scans can be initialized for execution according to the imaging parameters that were automatically recalled from the initial imaging session. The automatic recall of the imaging parameters can thus avoid extensive imaging parameters inputting by a clinician and also reduce the possibility of operator error.

The process may include, at 240, controlling the imaging (e.g., MRI) system to perform the treatment-day scans according to the recalled imaging parameters. For example, a sequence of scan planes, contrast types, etc., can be automatically and sequentially executed by the imaging system to provide imaging data analogous to the initial imaging session but capturing the patient's current anatomy.

Treatment-day scans may be performed to confirm the location of targets for radiation therapy as well as other anatomical structures that may have prescription limits on the amount of radiation they may receive (herein referred to as "organs at risk" (OARs). For example, in some cases the patient may undergo weight changes between treatment day and when they were initially scanned for generating the initial radiation treatment plan. Such changes in weight (or any other biological changes such as bladder or rectum fullness, inflammation, surgeries, etc.) can result in changes in shape and/or location of relevant anatomical structures of patient. With the treatment-day scans, the system can then proceed to perform or facilitate reoptimization or generation of radiation treatment plan(s) based on current patient conditions.

The process may include, at 250, automatically generating at least one reoptimized radiation treatment plan. In certain embodiments relating to treatment plan generation/reoptimization, "automatically" means that the generation of reoptimized radiation treatment plan(s) can occur prior to any assessment of the original treatment plan. For example, rather than a clinician determining that, based on treatment-day scans, reoptimization of the radiation treatment plan needs to be done, the system can automatically generate reoptimized radiation treatment plans based on the treatment-day scans. In some embodiments, the automatic generation can occur immediately (or with a small delay) when the treatment-day scanning is completed. However, it contemplated that in some systems there may be delays provided for various reasons. Accordingly, it is contemplated that while some embodiments of the present disclosure may have essentially zero or small delays (e.g., 1 second to perhaps 30 seconds), other embodiments may have delays up to several minutes, but nevertheless occur prior to assessment of the original treatment plan. Such longer days may not be optimal for rapid patient treatment but may be utilized in certain embodiments of the disclosed systems.

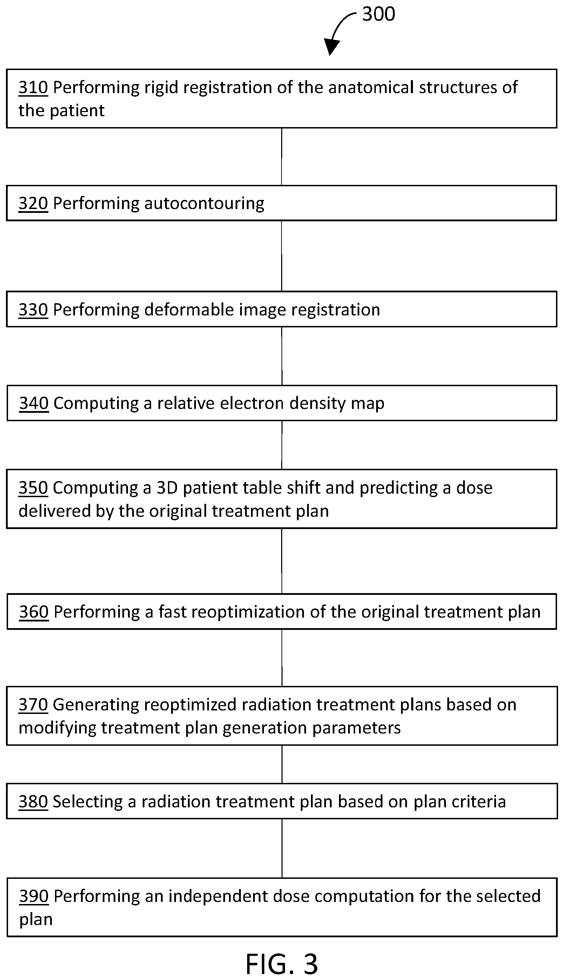

Further explanation and details of various embodiments of the system generating reoptimized radiation treatment plans are provided below with reference to Figure 3. Also, any number of reoptimized radiation treatment plans can be generated by the system with the different plans having different constraints or utilizing different methods. In this way, multiple reoptimized radiation treatment plans can be provided and the best one (i.e., one most completely meeting the radiation prescription) can be selected either automatically by the system or by a user.

Processes consistent with the present disclosure may include (at step 260 in Figure 2), displaying predicted doses to anatomical structures of the patient based on the reoptimized radiation treatment plane(s). In certain embodiments, the predicted doses may be automatically displayed without requiring any clinician input. The predicted doses can be provided for display at control console 110 and/or any of the remote computers 170. The displayed predicted doses can be in the form of, for example, heat maps, dose volume histograms, bar charts, etc. Predicted doses can include calculated quantities for what radiation doses will be delivered to various targets or other patient anatomy based on the corresponding imaging and the radiation delivery system. The predicted doses can be calculated, for example, utilizing methods such as Monte Carlo, pencil beam, convolution/superposition, discrete ordinates/Boltzmann transport, or any combination of the above.

The system can also store "prescription doses" that may include a list of targets and/or other patient anatomy (e.g., OARs) and an amount of radiation that the patient's physicians have determined should be delivered to each and optionally including an amount of tissue volume that will receive the radiation. The prescription doses can specify ranges, lower bounds, and/or upper bounds for such radiation doses. Such prescription doses can thus serve as a goal for radiation treatment plan reoptimization. This allows for acceptable variation in the acceptable treatment plan criteria considering the variations expected in the day-to-day changes in the patient.

Additionally, certain embodiments of the present disclosure can calculate and display prescription doses and/or predicted doses based on the original radiation treatment plan. In this way, both the newly generated reoptimized radiation treatment plan and (optionally) an original treatment plan can be compared to facilitate selection by clinicians, or by the system itself comparing plans, to determine which best meets the prescription.

The process may include, at 270, the system determining whether the provided treatment plan(s) (e.g., any of the original treatment plan and the generated reoptimized treatment plan(s)) are acceptable. For example, this can include the system performing comparisons with the predicted doses and the prescribed doses. If all prescriptions are met with the original treatment plan or the generated reoptimized treatment plan(s) then one (e.g., the best one) may be designated in the system as acceptable. If all prescriptions are not met, or it is determined that the provided radiation treatment plan(s) are not acceptable for any reason, then the process can provide for additional plan reoptimization as described below.

The process may include, at 280, providing for additional plan reoptimization and approvals. For example, in certain embodiments, the system can allow for a clinician to modify computer-generated contours of the patient anatomy (i.e., "auto" contours) to modify the predicted dose. In other embodiments, a relative electron density map can be modified, thereby changing the reoptimization. Details of these and further embodiments are described further below with reference to Figure 3.

When a satisfactory reoptimized radiation treatment plan has been generated and approved by the requisite personnel or determined by the system to meet the prescription, treatment may commence as described at step 290, controlling a radiation therapy device to deliver radiation according to a selected radiation treatment plan. In some embodiments, the selected radiation treatment plan can be automatically delivered by the system, for example, as soon as a sufficiently good radiation treatment plan is available. In other embodiments, the selected radiation treatment plan can be delivered by the system in conjunction with commands originating from the control console (e.g., pressing a button to start treatment).

As previously mentioned, an imaging system, such as an MRI, can be configured to perform imaging during treatment by performing one or more of 2D imaging, multiplanar 2D imaging, 3D imaging, 4D imaging, and real-time 4D volumetric imaging, for example, based on previously recalled imaging parameters. In certain embodiments, controlling of the radiation therapy device can be further based on the imaging. Such dynamic controlling of the radiation delivery can account for patient motion and/or changes in patient anatomy. In certain embodiments, a collimating system, a rotating gantry supporting a radiation source, a configuration of a robotic arm supporting a radiation source, etc., can be adjusted to adaptively deliver radiation based on ongoing analysis of the imaging. In other embodiments, radiation delivery can be suspended for analysis and the subsequent resumption of delivery can be adjusted based on the imaging.

Additionally, certain embodiments of the present disclosure can automatically calculate and display the data and/or information for controlling the radiation therapy device based on the imaging. Examples of such data and/or information can include the imaging dimensionality, imaging parameters, tissue-tracking boundaries type and generation parameters, and the tissue-tracking algorithm parameters. The imaging dimensionality can indicate whether an image is 2D, 3D, real-time 40 volumetric, etc. An example of a tissue-tracking algorithm parameter can include denoising. Examples of tissue tracking boundaries type can include specifying whether it is a target, OAR, a boundary (e.g., an expanded region around the boundary where radiation may permissibly be delivered), boundary generation parameters can specify various tools to make a boundary, such as a free-drawing line, or a movable brush region that can expand/contract an existing boundary by pushing the boundary to not be inside the brush region.

Figure 3 is a flowchart of an exemplary software-implemented process for automatic radiation treatment plan reoptimization (e.g., step 250 in Figure 2). Process 300 describes one example of calculations that can be formed to generate and/or select a reoptimized radiation treatment plan. The elements in process 300 need not be executed in the order shown nor are all elements necessary in all embodiments.

Process 300 may include, at 310, performing a rigid registration of the anatomical structures of the patient. A rigid registration can include determining a shift (e.g., a patient couch shift) that improves the agreement between the current patient position and where the patient should be (e.g., locating a treatment target at isocenter). Determination of the shift can be calculated by the system utilizing image analysis techniques such as image differencing, image spectral cross-correlation, etc.

The process may include, at 320, performing autocontouring of the anatomical structures of the patient in the treatment-day imaging. Autocontouring can include the system determining edges around various anatomical structures in the patient images. These edges (or contours) thus define in 2D (or 3D) the predicted extents of the anatomical structures. Rather than a clinician having to view multiple frames of an image and manually drawing for determining contours, the system can be configured to determine (or autocontour) the anatomy by any number of methods. One exemplary method can include gradient analysis where gradients in the image are assumed to represent changes in the physical structure of the patient, e.g., a boundary between a tumor and healthy tissue.

The process may include, at 330, performing deformable image registration. Deformable image registration allows for nonrigid changes in patient anatomy when performing registration of the patient. For example, a shape of a tumor in a current image may be elongated relative to the shape in a prior image. The differences between pixels or voxels can be weighted to provide a best shift refinement such that despite the deformed image, the computed shift results in the best positioning of the patient for treatment. In other examples, deformable image registration can be used for a best alignment of sequential images that may have changed due deformations. For example, to construct a 4D patient volume for display or radiation treatment planning purposes, elements of a current volumetric image can be deformed to determine a shift to best align with a prior image.

The process may include, at 340, computing a relative electron density map. A relative electron density map (RED) can be used in generating a reoptimized radiation treatment plan to provide a more accurate dose computation as methods of ionizing radiation dose computation for megavoltage photon beams are most accurate when this information can be provided. A RED can be generated by, for example, setting segmented anatomy to known RED values (known as a RED structure override), applying deformable image registration to a RED derived from the patient or a template of a similar patient, by generating a synthetic CT from the MR image of the patient, or a combination of these methods.

The process may include, at 350, computing a 3D patient table shift and/or calculating predicted doses to the anatomical structures. As previously explained, various registration techniques can result in a determination for how the system needs to adjust the patient to locate them in the proper position for treatment. The calculated patient table shift can be any combination of X, Y, and Z shifts and rotations. In other embodiments, the calculations and/or shifting can be performed in 2D or 1D, though generally such shifts can be expressed as a 3D shift even if a shift in a particular direction is unneeded. Also, the predicted doses to anatomical structures can be calculated as described herein and utilized during any of the reoptimization processes disclosed herein.

The process may include, at 360, performing a fast reoptimization of the original radiation treatment plan, or an optimization of the beam intensities of a previously existing treatment plan. A "fast" reoptimization can utilize existing beam shapes and beam angles, which were previously computed to predict the dose that would be delivered by the prior radiation treatment plan. In other implementations, a "full" reoptimization can be performed that can include computing beamlets, performing fluence map optimization, and sequencing and reoptimization of an MLC leaf positions. Fast reoptimization thereby can have a reduced computational overhead and/or take less time to complete as compared to a full reoptimization.

The process may include, at 370, generating one or more reoptimized radiation treatment plans based on modifying treatment plan generation parameters. The system can be configured to receive input changing one or more features of the at least one reoptimized radiation treatment plan. In some cases, better realization of the radiation prescription can be achieved by changing one or more of the following features: the position of the patient couch, contoured anatomical structures, RED structure override values, or optimization objective functions and constraints.
Examples of optimization objective functions and constraints can include penalty functions or operators such as >, ≥,<,≤, or = for a dose volume objective and a weight for this objective, in addition to min, max, mean, and median objectives. The system can be configured to then automatically recalculate a reoptimized radiation treatment plan based on the changed features.

While the inputs that change a radiation treatment plan can be provided based on user input (e.g., using a graphical interface to change a contour, entering a new value for a target dose or tolerance), in some embodiments, such changes can be automatically implemented by the system software to generate a number of reoptimized radiation treatment plans. For example, one embodiment can have the automatic generating of reoptimized radiation treatment plan(s) include generating reoptimized radiation treatment plans based on modifying treatment plan generation parameters. The generating of the reoptimized radiation treatment plan(s) can stop when one of the reoptimized treatment plans meets a treatment prescription or when a predetermined number of reoptimized radiation treatment plans are generated.

As an example of how parameters can be varied to generate different radiation treatment plans, first the system can compare the predicted statistics (e.g., doses to anatomical structures) to the prescription. Then, for example, if a predicted dose is too low, a weighting, importance, or power (if using a power function penalty) can be increased in the objective function that is trying to deliver the prescribed dose to the target volume. As another example, if a critical organ is being overdosed, the weight, importance, or power of the objective attempting to spare the organs can be increased. Such optimizations can be performed in an iterative fashion to reach the target prescription or the closest possible convergence.

As mentioned above, any number of such reoptimized radiation treatment plans can be generated, for example, one, two, three, five, ten, etc. This predetermined number can be set or modified by a user as desired or can be hardcoded into the software. In some embodiments, the radiation treatment plans can be generated sequentially (i.e., one finishes and then is checked against the prescription before starting another). In other embodiments, the radiation treatment plans can be generated in parallel. In some embodiments, the system can stop plan generation when it is determined that one of the plans meets the prescription. For example, five radiation treatment plans can be started and if the third plan is completed and determined to meet the prescription then any uncompleted plans in progress may be ceased. Such implementations can have the benefit of reducing unnecessary computational overhead and further reducing the time required to commence treatment.

The process may include, at 380, selecting a reoptimized radiation treatment plan based on plan criteria (e.g., the prescription). In some embodiments, the software can be configured to allow a user to select from any number of reoptimized radiation treatment plans that were calculated as described herein. For example, while in some embodiments there may only be one (best) plan generated, in other embodiments there may be multiple plans generated and some may be different than others, but all still meet the prescription. The clinician can then have the option to select from these reoptimized radiation treatment plans. In other embodiments, the best radiation treatment plan can be selected by the system based on an algorithm, for example, considering the prescription and optionally the relative importance of individual criteria in the prescription.

The process may include, at 390, performing an independent dose computation for the selected plan. In some embodiments, radiation planning quality assurance can include utilizing different independent (i.e., using different algorithms and methods) dose calculation software than was used to evaluate validity of creation/selection of the reoptimized radiation treatment. The re-computation of dose with an independent algorithm can be performed to ensure that independent methods agree on the dose values for safety assurance. For example, the independent dose computation can utilize verified software specifically entrusted to accurately compute the dose that will be delivered by the selected reoptimized radiation therapy plan. The independent dose computation can be performed on a different software module of the disclosed system or can be provided to an outside computer that will perform the independent dose computation and return results for storage and display to users of the disclosed system.

As described herein, numerous embodiments include comparisons between prescription doses and the predicted doses for a particular radiation treatment plan. While such comparisons can be completely internal to the disclosed software (i.e., never seen by a user), in some embodiments the system can be configured for computation and displaying of prescription doses and/or criteria to the anatomical structures concurrently with the displaying of the predicted doses. For example, there can be a table having columns representing prescription and predicted doses for radiation treatment plans. The table can have rows that can further break down the displayed doses by anatomical structure for a total dose to a patient. Also, some embodiments can include highlighting or other visual indicia such as color coding to indicate whether a predicted dose is above, below, or within the range or parameter set forth by the prescription.

In certain circumstances, doctors and clinicians may wish to revise aspects of the original treatment plan or one of the reoptimized treatment plans. For example, they may wish to shift the patient couch or revise the contours that have been drawn around the targets or other organs. As described further below, the disclosed software can provide for additional treatment plan reoptimization by enabling clinicians to view aspects of one or more radiation treatment plans and modify them or control the software to perform reoptimization based on updated parameters. This providing for additional treatment plan reoptimization can thus include generation of a new reoptimized radiation treatment plan or modification of one of the automatically generated reoptimized radiation treatment plane(s). The viewing/modifying of reoptimized radiation treatment plans can be facilitated by an interactive graphical user interface provided at control console or any remote computer. Such interfaces can permit changing of radiation treatment plan parameters, anatomical contours, the patient prescription, etc.

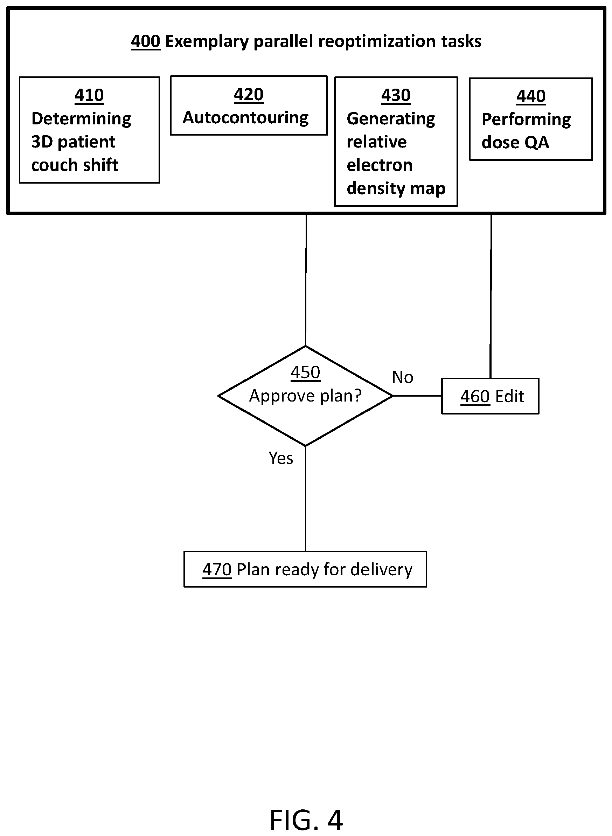

Figure 4 depicts examples of reoptimization tasks 400 that can be performed in parallel to speed the creation of a final reoptimized radiation treatment plan. The present disclosure contemplates that the software and system can provide for additional treatment plan reoptimization by enabling clinicians to perform different reoptimization tasks simultaneously. While the particular reoptimization tasks described herein can be part of the parallel operations enabled by the software, the present disclosure also contemplates general systems and software allowing for the parallelization of various tasks related to radiotherapy planning treatment.

Reoptimization processes 400 can include, at 410, software receiving input from a first clinician regarding a 3D patient couch shift. This can include not just modifying the coordinates or orientation of the patient couch but may also include analysis of the effect of potential couch shifts on the quality of image registration (e.g., based on quantitative metrics such as image correlations, predicted dose, etc., to determine the efficacy of the patient couch shift).

At 420, the software can receive input from a second clinician tasked with adjusting contours around patient anatomy. This can include, for example, modifying contouring performed by the software to edit/correct displayed contours to better match the anatomical structures depicted in patient images. Examples of such modifications can include adding/removing portions of an area bounded by a contour. Such editing can be performed in a 2D image or in a number of 2D images that together form part of a 3D patient volume. In some embodiments, the providing for additional treatment plan reoptimization includes enabling multiple clinicians to perform autocontouring simultaneously. For example, there may be 20 anatomical structures that are contoured and they may be divided amongst two (or more) remote computers for clinicians to simultaneously edit their assigned contours. The contours can be stored at the server and may be continuously updated based on changes to individual contours that are made at remote computers are transmitted to the server.

At 430, the software can receive input from a third clinician to generate a relative electron density map. A relative electron density map can be displayed and edited by the third clinician to adjust the values to better represent the particular matter in the image (e.g., adjusting the electron densities to be proper for regions considered to be bone, fat, cancerous tissue, etc.). Adjustments to the relative electron density map can be reflected in predicted dose calculations for the various patient anatomical structures.

At 440, the software can receive input from a fourth clinician tasked with performing radiation dose QA. Based on the radiation treatment plan (e.g., being generated/modified in parallel by the above clinicians) the software can calculate predicted radiation dose to anatomical structures and display them for the evaluating clinician. The clinician can then determine whether the predicted dose is being accurately calculated, for example, by comparing with an independent dose calculation for the radiation treatment plan. The clinician can then electronically advise the other clinicians performing the reoptimization tasks such that the radiation treatment plan passes or fails the required quality assurance checks.

The software provides for additional treatment plan reoptimization by enabling clinicians to attempt different reoptimization strategies simultaneously. For example, IMRT treatment plan objectives can be set or modified with different strategies by different clinicians for both target coverage and sparing of critical organs to meet the prescription. For example, the system can be configured to receive and process inputs from one clinician for exploring higher importance weights for a critical organ while other inputs from another clinician can explore lower importance weights for a target, so both approaches can be efficiently compared. If the prescription is unable to be met, then one strategy may be to change/relax the prescription to allow treatment. Another strategy may be to delay therapy until the patient's organs geometry is adjusted. For example, the patient may move around, such as to settle/adjust fluid or waste distributions and return to the patient couch. Or, the patient may return for treatment on a different day, such as to allow for digestion of food causing organ displacement.

At 450, the software can require approval of certain changes or a final plan approval. Thus, in some embodiments, the software can be configured to receive input from a clinician approving the selected radiation treatment plan. For example, a clinician can be required to approve use of the initial radiation treatment plan, the reoptimized radiation treatment plan that has been selected or, as shown in Figure 4, approval of a plan that has been reoptimized through the parallel interface described herein. Whether the plan is ready for delivery or requires further editing can be determined based on the approval.

At 460, if approval is not given, or specifically denied, then system can permit further editing of one or more aspects of the available radiation treatment plans (e.g., original or reoptimized) according to any of the embodiments of the present disclosure. For example, any of the above elements of 410-440, or others, can be edited or re-executed based on user input. In some embodiments, the system can display one or more images from the available radiation treatment plans at one or more of the remote computers. The system can receive user input from any of these remote computers approving the images and/or the radiation treatment plane(s). Alternatively, the system can receive user input modifying, or suggesting a modification of, one or more of the reoptimized radiation treatment plane(s). For example, the system can be configured to cause modification of the radiation treatment plan based on user input such as changing contours around anatomical structures, prescription parameters, locations of imaging planes, etc.

For the example of moving an imaging plane, user input can specify that an imaging plane should be moved to a certain location such that a particular anatomical structure (or portion thereof) is (or is not) captured in the images that will be acquired during treatment or used for radiation treatment plan generation. In some embodiments, the system can update the reoptimized radiation treatment plans(s) based on the user input. Because radiation treatment plans are generated based on available imaging (e.g., the targets and other anatomical structures are particular to a given image in a chosen plane) any user input that changes/moves such imaging planes therefore can be the basis of an updated reoptimized radiation treatment plan. For example, suppose a given image plane through the patient shows the target but not a nearby anatomical structure that the prescription wants to avoid delivering radiation to. A reoptimized radiation treatment plan may then determine treatment parameters such as number of beams, their delivery vectors, etc., such that the target receives radiation, but because the structure to be avoided is not in the image, the reoptimized radiation treatment plan may not necessarily account for the nearby anatomical structure. However, upon receiving input moving the imaging plane to another location, it is possible that the anatomical structure is now in the imaging plane. Accordingly, the system can reoptimize the radiation treatment plan and presumably find different solutions for the delivery of radiation to the target - now taking into account the nearby anatomical structure where radiation delivery is to be avoided. Similarly, radiation treatment plans can include gating parameters, and changing the imaging plane for gating can result in an altered treatment plan and delivery.

In other embodiments, imaging parameters can be modified by user input with examples of such imaging parameters including patient orientation, field-of-view, number of imaging planes, what planes to track, plane locations, and imaging resolution. Changing any combination of the imaging planes/parameters can thus similarly serve as the basis for the system modifying a reoptimize radiation treatment plan as explained above.

In other embodiments, the system can be configured to receive user input modifying one or more of tracking parameters, dose parameters, structure shapes, or boundary parameters utilized by the system when controlling the radiation therapy device to deliver the radiation. Examples of tracking parameters can include specifying a degree of how much anatomical structures deform, whether an anatomical structure is stationary or moving, a field of view for the imaging, and imaging parameters (such as described above). Examples of dose parameters can include the prescription whereby the prescription includes limits and ranges of acceptable radiation delivery. Structure shapes can include contours around anatomical structures, for example, contours that specify the gross tumor volume (GTV) or any other contours that have been generated around anatomical structures of interest. Boundary parameters can include specifying regions around anatomical structures that may be used for planning purposes, sometimes referred to as a planning target volume (PTV). For example, a boundary perimeter for a given anatomical structure can be set to include 5% more area or volume resulting in the system generating a slightly expanded perimeter around the identified anatomical structure to allow for a greater permissible region that can receive radiation.

At 470, if approval is given, the system may designate the selected radiation treatment plan as ready for delivery. This can include, for example, automatically delivering the radiation treatment plan as described herein to facilitate the system's ability to deliver radiation treatment in a minimum amount of time.

The present disclosure contemplates that the calculations disclosed in the embodiments herein may be performed in a number of ways, applying the same concepts taught herein, and that such calculations are equivalent to the embodiments disclosed.

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" (or "computer readable medium") refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" (or "computer readable signal") refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

The subject matter described herein can be embodied in systems, apparatus, methods, computer programs and/or articles depending on the desired configuration. Any methods or the logic flows depicted in the accompanying figures and/or described herein do not necessarily require the particular order shown, or sequential order, to achieve desirable results. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. The implementations described above can be directed to various combinations and subcombinations of the disclosed features and/or combinations and subcombinations of further features noted above. Furthermore, above described advantages are not intended to limit the application of any issued claims to processes and structures accomplishing any or all of the advantages.

Additionally, section headings shall not limit or characterize the invention(s) set out in any claims that may issue from this disclosure. Further, the description of a technology in the "Background" is not to be construed as an admission that technology is prior art to any invention(s) in this disclosure. Neither is the "Summary" to be considered as a characterization of the invention(s) set forth in issued claims. Furthermore, any reference to this disclosure in general or use of the word "invention" in the singular is not intended to imply any limitation on the scope of the claims set forth below. Multiple inventions may be set forth according to the limitations of the multiple claims issuing from this disclosure, and such claims accordingly define the invention(s), that are protected thereby.

## Claims

1. A non-transitory, machine-readable medium storing instructions which, when executed by at least one programmable processor, cause the at least one programmable processor to perform operations comprising:
automatically recalling imaging parameters from computer memory for controlling an MRI system to perform treatment-day scans of a patient on a treatment couch prior to treatment;
sensing a treatment-day scanning trigger being sensing that the patient is positioned at isocenter or a sensor indicating a door to a treatment room is closed, automatically initializing the treatment-day scans based on sensing the treatment-day scanning trigger,
controlling the MRI system to perform the treatment-day scans according to the recalled imaging parameters;
automatically generating at least one reoptimized radiation treatment plan;
displaying predicted doses to anatomical structures of the patient based on the at least one reoptimized radiation treatment plan; and
controlling a radiation therapy device to deliver radiation according to a selected radiation treatment plan.

2. The machine-readable medium of claim 1, wherein the automatic initializing of the treatment-day scans is based on a sensor indicating a door to a treatment room is closed.

3. The machine-readable medium of claim 1, the automatically generating of the at least one reoptimized radiation treatment plan further comprising:
performing autocontouring of the anatomical structures of the patient;
computing a relative electron density map;
computing a 3D patient table shift; and
calculating the predicted doses to the anatomical structures.

4. The machine-readable medium of claim 1, the automatically generating of the at least one reoptimized radiation treatment plan further comprising:
generating reoptimized radiation treatment plans based on modifying treatment plan generation parameters, the generating stopping when one of the reoptimized treatment plans meets a treatment prescription or when a predetermined number of reoptimized radiation treatment plans are generated.

5. The machine-readable medium of claim 1, the operations further comprising:
receiving input changing one or more features of the at least one reoptimized radiation treatment plan; and
automatically recalculating the at least one reoptimized radiation treatment plan based on the changed features.

6. The machine-readable medium of claim 1, the operations further comprising:
automatically calculating and displaying information for controlling the radiation therapy device based on the imaging.

7. The machine-readable medium of claim 1, wherein the information comprises one or more of: imaging dimensionality, imaging parameters, tissue-tracking boundaries type and generation parameters, and tissue-tracking algorithm parameters.

8. The machine-readable medium of claim 1, the operations further comprising providing for additional treatment plan reoptimization.

9. The machine-readable medium of claim 8, wherein providing for additional treatment plan reoptimization includes generation of a new reoptimized radiation treatment plan, or modification of one of the automatically generated reoptimized radiation treatment plane(s).

10. The machine-readable medium of claim 8, wherein providing for additional treatment plan reoptimization includes enabling clinicians to perform different reoptimization tasks simultaneously.

11. The machine-readable medium of claim 8, wherein providing for additional treatment plan reoptimization includes enabling multiple clinicians to perform autocontouring simultaneously.

12. The machine-readable medium of claim 8, wherein providing for additional treatment plan reoptimization includes enabling clinicians to attempt different reoptimization strategies simultaneously.

13. The machine-readable medium of claim 8, wherein providing for additional treatment plan reoptimization includes the system receiving user input modifying one or more of the reoptimized radiation treatment plane(s).

14. The machine-readable medium of claim 13, the operations further comprising:
moving an imaging plane included in the one or more of the reoptimized radiation treatment plan(s) based on the user input; and
updating the one or more of the reoptimized radiation treatment plan(s) based on a new location of the imaging plane.

15. The machine-readable medium of claim 1, further comprising:
receiving user input modifying one or more of tracking parameters, dose parameters, structure shapes, or boundary parameters utilized by the system when controlling the radiation therapy device to deliver the radiation.

## Patentansprüche

1. Nicht-transitorisches, maschinenlesbares Medium, das Anweisungen speichert, die, wenn sie von mindestens einem programmierbaren Prozessor ausgeführt werden, den mindestens einen programmierbaren Prozessor veranlassen, Operationen auszuführen, umfassend:
automatisches Abrufen von Bildgebungsparametern aus dem Computerspeicher zum Steuern eines MRT-Systems, um Behandlungstags-Scans eines Patienten auf einer Behandlungsliege vor der Behandlung durchzuführen;
Erfassen eines Behandlungstags-Scan-Triggers, wobei das Erfassen darin besteht, dass der Patient am Isozentrum positioniert ist oder ein Sensor anzeigt, dass eine Tür zu einem Behandlungsraum geschlossen ist, automatisches Initialisieren der Behandlungstags-Scans basierend auf dem Erfassen des Behandlungstags-Scan-Triggers,
Steuern des MRT-Systems, um die Behandlungstags-Scans gemäß den abgerufenen Bildgebungsparametern durchzuführen;
automatisches Erzeugen mindestens eines reoptimierter Strahlenbehandlungsplans;
Anzeigen vorhergesagter Dosen für anatomische Strukturen des Patienten basierend auf dem mindestens einen reoptimierter Strahlenbehandlungsplan; und
Steuern einer Strahlentherapievorrichtung, um Strahlung gemäß einem ausgewählten Strahlenbehandlungsplan abzugeben.

2. Maschinenlesbares Medium nach Anspruch 1, wobei das automatische Initialisieren der Behandlungstags-Scans auf einem Sensor basiert, der anzeigt, dass eine Tür zu einem Behandlungsraum geschlossen ist.

3. Maschinenlesbares Medium nach Anspruch 1, wobei das automatische Erzeugen des mindestens einen reoptimierter Strahlenbehandlungsplans ferner umfassend:
Autokonturieren der anatomischen Strukturen des Patienten;
Berechnen einer Elektronendichtekarte;
Berechnen einer 3D-Patiententischverschiebung; und
Errechnen der vorhergesagten Dosen für die anatomischen Strukturen.

4. Maschinenlesbares Medium nach Anspruch 1, wobei das automatische Erzeugen des mindestens einen reoptimierter Strahlenbehandlungsplans ferner umfassend:
Erzeugen reoptimierter Strahlenbehandlungspläne basierend auf dem Modifizieren von Behandlungsplanerzeugungsparametern, wobei das Erzeugen stoppt, wenn eines von den reoptimierter Behandlungsplänen eine Bestrahlungsvorschrift erfüllt oder wenn eine vorbestimmte Anzahl reoptimierter Strahlenbehandlungspläne erzeugt worden ist.

5. Maschinenlesbares Medium nach Anspruch 1, wobei die Operationen ferner umfassend:
Empfangen einer Eingabe, die ein oder mehrere Merkmale des mindestens einen reoptimierter Strahlenbehandlungsplans ändert; und
automatisches Neuberechnen des mindestens einen reoptimierter Strahlenbehandlungsplans basierend auf den geänderten Merkmalen.

6. Maschinenlesbares Medium nach Anspruch 1, wobei die Operationen ferner umfassend:
automatisches Errechnen und Anzeigen von Informationen zum Steuern der Strahlentherapievorrichtung basierend auf der Bildgebung.

7. Maschinenlesbares Medium nach Anspruch 1, wobei die Informationen eines oder mehrere von Folgendem umfassen: Bildgebungsdimensionalität, Bildgebungsparameter, Typ und Erzeugungsparameter von Gewebeverfolgungsgrenzen sowie Parameter des Gewebeverfolgungsalgorithmus.

8. Maschinenlesbares Medium nach Anspruch 1, wobei die Operationen ferner das Bereitstellen für eine zusätzliche Reoptimierung des Behandlungsplans umfassen.

9. Maschinenlesbares Medium nach Anspruch 8, wobei das Bereitstellen für eine zusätzliche Reoptimierung des Behandlungsplans das Erzeugen eines neuen reoptimierter Strahlenbehandlungsplans oder die Modifikation eines der automatisch erzeugten reoptimierter Strahlenbehandlungsplan(s) beinhaltet.

10. Maschinenlesbares Medium nach Anspruch 8, wobei das Bereitstellen für eine zusätzliche Reoptimierung des Behandlungsplans das Ermöglichen beinhaltet, dass Kliniker verschiedene Reoptimierungsaufgaben gleichzeitig ausführen.

11. Maschinenlesbares Medium nach Anspruch 8, wobei das Bereitstellen für eine zusätzliche Reoptimierung des Behandlungsplans das Ermöglichen beinhaltet, dass mehrere Kliniker gleichzeitig Autokonturieren durchführen.

12. Maschinenlesbares Medium nach Anspruch 8, wobei das Bereitstellen für eine zusätzliche Reoptimierung des Behandlungsplans das Ermöglichen beinhaltet, dass Kliniker verschiedene Reoptimierungsstrategien gleichzeitig versuchen.

13. Maschinenlesbares Medium nach Anspruch 8, wobei das Bereitstellen für eine zusätzliche Reoptimierung des Behandlungsplans beinhaltet, dass das System eine Benutzereingabe empfängt, die einen oder mehrere der reoptimierter Strahlenbehandlungsplan(s) modifiziert.

14. Maschinenlesbares Medium nach Anspruch 13, wobei die Operationen ferner umfassend:
Verschieben einer Bildgebungsebene, die in dem einen oder den mehreren der reoptimierter Strahlenbehandlungsplan(s) enthalten ist, basierend auf der Benutzereingabe; und
Aktualisieren des einen oder der mehreren der reoptimierter Strahlenbehandlungsplan(s) basierend auf einer neuen Position der Bildgebungsebene.

15. Maschinenlesbares Medium nach Anspruch 1, ferner umfassend: Empfangen einer Benutzereingabe, die einen oder mehrere von Verfolgungsparametern, Dosisparametern, Strukturformen oder Grenzparametern modifiziert, die vom System beim Steuern der Strahlentherapievorrichtung zur Abgabe der Strahlung verwendet werden.

## Revendications

1. Support non transitoire lisible par machine stockant des instructions qui, lorsqu'elles sont exécutées par au moins un processeur programmable, amènent l'au moins un processeur programmable à effectuer des opérations comprenant :
le rappel de manière automatique de paramètres d'imagerie à partir de la mémoire de l'ordinateur pour commander à un système d'IRM d'effectuer des balayages le jour du traitement d'un patient sur un divan de traitement avant le traitement ;
la détection d'un déclencheur de balayage le jour de traitement, notamment la détection que le patient est positionné à l'isocentre ou la fermeture d'une porte d'une salle de traitement indiquée par un capteur, initialisant automatiquement les balayages le jour de traitement sur la base de la détection du déclencheur de balayage le jour de traitement,
la commande au système d'IRM d'effectuer les balayages le jour de traitement en fonction des paramètres d'imagerie rappelés ;
la génération de manière automatique d'au moins un plan de traitement de radiothérapie réoptimisé ;
l'affichage des doses prévues pour les structures anatomiques du patient sur la base de l'au moins un plan de traitement de radiothérapie réoptimisé ; et
la commande à un dispositif de radiothérapie d'administrer des rayonnements en fonction d'un plan de traitement de radiothérapie sélectionné.

2. Support lisible par machine selon la revendication 1, dans lequel l'initialisation de manière automatique des balayages le jour de traitement est basée sur la fermeture d'une porte d'une salle de traitement indiquée par un capteur.

3. Support lisible par machine selon la revendication 1, la génération de manière automatique de l'au moins un plan de traitement de radiothérapie réoptimisé comprenant en outre :
la réalisation d'un autocontourage des structures anatomiques du patient ;
le calcul d'une carte de densité électronique relative ;
le calcul d'un déplacement de table de patient 3D ; et
le calcul des doses prévues pour les structures anatomiques.

4. Support lisible par machine selon la revendication 1, la génération de manière automatique de l'au moins un plan de traitement de radiothérapie réoptimisé comprenant en outre :
la génération de plans de traitement de radiothérapie réoptimisés sur la base de la modification de paramètres de génération de plans de traitement, la génération s'arrêtant lorsque l'un des plans de traitement réoptimisés répond à une prescription de traitement ou lorsqu'un nombre prédéterminé de plans de traitement de radiothérapie réoptimisés ont été générée.

5. Support lisible par machine selon la revendication 1, les opérations comprenant en outre :
la réception d'informations modifiant une ou plusieurs caractéristiques de l'au moins un plan de traitement de radiothérapie réoptimisé ; et
le recalcul de manière automatique de l'au moins un plan de traitement de radiothérapie réoptimisé sur la base des caractéristiques modifiées.

6. Support lisible par machine selon la revendication 1, les opérations comprenant en outre :
le calcul et l'affichage de manière automatique des informations nécessaires à la commande du dispositif de radiothérapie sur la base de l'imagerie.

7. Support lisible par machine selon la revendication 1, dans lequel les informations comprennent un ou plusieurs des éléments suivants : la dimensionnalité de l'imagerie, les paramètres d'imagerie, le type et les paramètres de génération de limites de suivi des tissus, et les paramètres de l'algorithme de suivi des tissus.

8. Support lisible par machine selon la revendication 1, les opérations comprenant en outre la possibilité d'une réoptimisation supplémentaire du plan de traitement.

9. Support lisible par machine selon la revendication 8, dans lequel la possibilité d'une réoptimisation supplémentaire du plan de traitement inclut la génération d'un nouveau plan de traitement de radiothérapie réoptimisé ou la modification d'un parmi le ou les plans de traitement de radiothérapie réoptimisés générés automatiquement.

10. Support lisible par machine selon la revendication 8, dans lequel la possibilité d'une réoptimisation supplémentaire du plan de traitement inclut la possibilité pour les cliniciens d'effectuer simultanément différentes tâches de réoptimisation.

11. Support lisible par machine selon la revendication 8, dans lequel la possibilité d'une réoptimisation supplémentaire du plan de traitement inclut la possibilité pour de multiples cliniciens d'effectuer simultanément un autocontourage.

12. Support lisible par machine selon la revendication 8, dans lequel la possibilité d'une réoptimisation supplémentaire du plan de traitement inclut la possibilité pour les cliniciens de tenter simultanément différentes stratégies de réoptimisation.

13. Support lisible par machine selon la revendication 8, dans lequel la possibilité d'une réoptimisation supplémentaire du plan de traitement inclut la réception par le système d'une entrée de l'utilisateur modifiant un ou plusieurs parmi le ou les plans de traitement de radiothérapie réoptimisés.

14. Support lisible par machine selon la revendication 13, les opérations comprenant en outre :
le déplacement d'un plan d'imagerie inclus dans le ou les plans parmi le ou les plans de traitement de radiothérapie réoptimisés sur la base de l'entrée de l'utilisateur ; et
la mise à jour du ou des plans parmi le ou les plans de traitement de radiothérapie réoptimisés sur la base d'un nouvel emplacement du plan d'imagerie.

15. Support lisible par machine selon la revendication 1, comprenant en outre :
la réception d'une entrée de l'utilisateur modifiant un ou plusieurs éléments parmi les paramètres de suivi, les paramètres de dose, les formes des structures ou les paramètres de limites utilisés par le système lors de la commande de l'administration des rayonnements par le dispositif de radiothérapie.
